(19) Europäisches Patentamt
European Patent Office
Office européen des brevets

(11) Numéro de publication: **0 514 288 B1**

(12) # FASCICULE DE BREVET EUROPEEN

(45) Date de publication de fascicule du brevet: **29.03.95**

(51) Int. Cl.[6]: **C07C 57/13**, C07C 51/12, C07C 69/593, C07C 67/37

(21) Numéro de dépôt: **92420119.7**

(22) Date de dépôt: **13.04.92**

(54) **Procédé de dicarbonylation linéaire de butènes difonctionnalisés.**

(30) Priorité: **17.05.91 FR 9106188**

(43) Date de publication de la demande:
**19.11.92 Bulletin 92/47**

(45) Mention de la délivrance du brevet:
**29.03.95 Bulletin 95/13**

(84) Etats contractants désignés:
**BE DE ES FR GB IT NL**

(56) Documents cités:
**EP-A- 0 347 340**
**EP-A- 0 395 545**

(73) Titulaire: **RHONE-POULENC CHIMIE**
**25, Ouai Paul Doumer**
**F-92408 Courbevoie Cédex (FR)**

(72) Inventeur: **Huser, Marc**
**65, rue Anatole France**
**F-69100 Villeurbanne (FR)**
Inventeur: **Mutez, Sylvain**
**33, rue de Combemore,**
**5, Hameau des Presles**
**F-69540 Irigny (FR)**
Inventeur: **Perron, Robert**
**La Pecolière**
**F-69390 Charly (FR)**

(74) Mandataire: **Vignally, Noel et al**
**RHONE-POULENC CHIMIE,**
**Direction de la Propriété Industrielle,**
**Centre de Recherches des Carrières,**
**B.P. 62**
**F-69192 Saint-Fons Cédex (FR)**

**EP 0 514 288 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé de dicarbonylation linéaire de butènes difonctionnalisés. Par dicarbonylation linéaire on entend la formation majoritaire de l'acide hexène-3 dioïque-1,6 et/ou de ses diesters alkyliques, par réaction du monoxyde de carbone, le cas échéant d'un alcool, sur au moins un butène disubstitué par des groupes hydroxy, alcoxy ou acyloxy.

Les diesters de l'acide hexène-3 dioïque-1,6 peuvent être hydrogénés en diesters de l'acide adipique correspondants qui peuvent alors être hydrolysés pour former l'acide adipique.

L'acide hexène-3 dioïque-1,6 peut être hydrogéné en acide adipique.

L'acide adipique, l'une des matières premières du nylon 66 est produit avec de forts tonnages et de ce seul fait, toute nouvelle voie d'accès à ce diacide et/ou à ses dérivés présente un intérêt de principe immédiatement perceptible.

Dans la demande de brevet européen EP-A-0 347 340 (correspondant au brevet US 4 925 973), il a été proposé un procédé catalytique de préparation de diesters de l'acide hexène-3 dioïque-1,6 par réaction du monoxyde de carbone et d'un alcool sur au moins un butène disubstitué par des groupes acyloxy. La dicarbonylation linéaire est effectuée en présence d'un catalyseur à base de palladium et d'un halogénure d'onium quaternaire d'un élément du groupe VB choisi parmi l'azote et le phosphore, ledit élément étant tétracoordonné à des atomes de carbone, l'azote pouvant être coordonné à 2 atomes de phosphore pentavalent, l'anion halogénure étant choisi parmi le chlorure et le bromure. Un tel procédé permet de réaliser la carbonylation dans des conditions de pression et de température acceptables à l'échelle industrielle, avec une sélectivité appréciable en produit dicarbonylé linéaire, les proportions de produit monocarbonylé et de produits dicarbonylés branchés étant minimes.

Dans la demande de brevet européen EP-A-0 395 545 il a été proposé un procédé catalytique de préparation de l'acide hexènedioïque-1,6 par réaction du monoxyde de carbone et de l'eau sur au moins un butène disubstitué par des groupes acyloxy. La dicarbonylation linéaire est effectuée également en présence d'un catalyseur à base de palladium et d'un chlorure d'onium quaternaire au sens indiqué ci-avant.

L'exemple 8 de cette demande réalisé avec du palladium métallique déposé sur charbon et en présence de chlorure de tétrabutylphosphonium ne conduit pas à des résultats entièrement satisfaisants.

Dans la demande de brevet européen EP-A-0 395 546 il a été notamment proposé un procédé catalytique de préparation de l'acide héxène-3 dioïque-1,6 par réaction du monoxyde de carbone et du butène-2 diol-1,4 et/ou du butène-1 diol-3,4.

La dicarbonylation linéaire est effectuée en présence d'un catalyseur à base de palladium et d'un chlorure d'onium quaternaire au sens précité.

L'exemple 21 de cette demande réalisé avec du palladium métallique déposé sur charbon en présence de chlorure de tétrabutylphosphonium ne conduit pas à des résultats pleinement satisfaisants.

Dans les demandes de brevet européen déposées respectivement sous les numéros 90/322 256, 90/322 257 et 90/322 258 il a été proposé de remplacer au moins partiellement l'halogènure d'onium au sens précité par le couple formé de certains halogénures minéraux et d'un solvant polaire aprotique basique.

Dans la demande de brevet français déposée sous le numéro 90/12042 il a été proposé un autre procédé catalytique de préparation de diesters de l'acide hexène-3 dioïque-1,6 par réaction du monoxyde de carbone sur un dialcoxy-1,2 butène-3 pris isolément ou en mélange avec un dialcoxy-1,4 butène-2.

La dicarbonylation linéaire est effectuée en présence d'un catalyseur à base de palladium et d'un chlorure ionique dont le cation est choisi dans le groupe constitué par les cations des métaux alcalins, les cations des métaux alcalino terreux et les cations d'onium quaternaire au sens précisé précédemment.

Toutefois l'ensemble de ces procédés catalytiques de dicarbonylation linéaire de butènes difonctionnalisés dont l'intérêt de principe n'est pas contesté présente au moins l'un des inconvénients suivants :

- certaines sources de palladium et, en particulier le palladium métallique déposé sur un support, ne présentent pas, dans certaines conditions, une activité pleinement satisfaisante.
- le système catalytique présente une stabilité insuffisante au cours du temps ce qui peut se traduire par l'observation de palladium précipité sur les parois et le fond du réacteur de carbonylation.

Il serait donc hautement souhaitable de disposer d'un procédé catalytique de dicarbonylation linéaire de butènes difonctionnalisés, dans lequel le système catalytique à base de palladium présenterait une stabilité améliorée et, le cas échéant, une activité catalytique améliorée.

La présente invention a donc pour objet un procédé de dicarbonylation linéaire de butènes difonctionnalisés en présence d'une quantité catalytiquement efficace de palladium et d'un chlorure d'onium quaternaire d'un élément du groupe VB choisi parmi l'azote et le phosphore, ledit élément étant tétracoordonné à des

atomes de carbone, l'azote pouvant être coordonné à 2 atomes de phosphore pentavalent, en phase liquide, à température élevée et sous une pression supérieure à la pression atmosphérique, caractérisé en ce que

a) le palladium est engagé au moins partiellement sous forme de palladium à l'état d'oxydation nul et
b) la réaction est conduite également en présence d'au moins une source de chlorure d'hydrogène.

Comme indiqué en tête du présent mémoire, l'une des matières premières utilisées dans le cadre du présent procédé est au moins un butène difonctionnalisé, c'est-à-dire disubstitué par des groupes hydroxy, alcoxy ou acyloxy, qu'il s'agisse de butène-2 substitués en positions 1 et 4 ou de butène-3 substitués en positions 1 et 2. Ces butènes difonctionnalisés peuvent être représentés par l'une ou l'autre des formules ci-après :

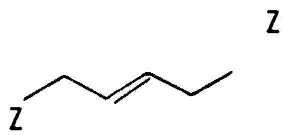
,
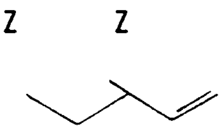

dans lesquelles Z représente un groupe -OH, -OR ou -O-(CO)-R ; R est un radical alkyle linéaire, branché ou cyclique qui comporte de 1 à 12 atomes de carbone et, de préférence de 1 à 4 atomes de carbone.

A titre d'exemples de butènes difonctionnalisés utiles dans le cadre du présent procédé, on peut citer :

- le butène-2 diol-1,4 le butène-3 diol-1,2 et leurs mélanges
- le diméthoxy-1,4 butène-2, le diméthoxy-1,2 butène-3 et leurs mélanges
- le diéthoxy-1,4 butène-2, le diéthoxy-1,2 butène-3 et leurs mélanges
- le diacétoxy-1,4 butène-2, le diacétoxy-1,2 butène-3 et leurs mélanges

Bien qu'il n'y ait pas d'obstacle de principe à choisir un mélange de diverses matières premières parmi lesdits butènes disubstitués, il s'avère en pratique plus intéressant de choisir des mélanges de diols, des mélanges de dialcoxybutènes ou des mélanges de diacyloxybutènes.

Il a en effet été constaté par la Demanderesse que la sélectivité en produit de dicarbonylation linéaire est sensiblement la même que l'on parte d'un butène-2 disubstitué en positions 1,4 ou d'un butène-3 disubstitué en positions 1,2, par un type de substituants donné.

A partir de butènediol(s) et de monoxyde de carbone, on obtiendra par la mise en oeuvre du procédé selon l'invention, comme produit majoritaire, l'acide héxène-3 dioïque-1,6.

A partir de dialcoxybutène(s) et de monoxyde de carbone, on obtiendra par la mise en oeuvre du procédé selon l'invention, comme produit majoritaire, l'hexène-3 dioate d'alkyle correspondant.

A partir de diacyloxybutène(s) de monoxyde de carbone et d'eau, on obtiendra par la mise en oeuvre du procédé selon l'invention, comme produit majoritaire, l'acide hexène-3 dioïque-1,6.

A partir de diacyloxybutène(s) de monoxyde de carbone, et d'un alcanol, on obtiendra par la mise en oeuvre du procédé selon l'invention, comme produits majoritaires l'acide hexène-3 dioïque-1,6 et ses esters alkyliques, dans des proportions variables selon les conditions de réaction.

Selon une caractéristique essentielle de la présente invention, la dicarbonylation est réalisée en présence de palladium, engagé au moins partiellement sous forme de palladium à l'état d'oxydation nul.

Pour ce faire, on peut recourir à du palladium métallique finement divisé ou à du palladium métallique déposé sur un support, tel les silices, les alumines, les silices-alumines, la zircone, le quartz, les argiles et les charbons actifs.

Du palladium déposé sur du charbon actif convient plus particulièrement à la mise en oeuvre du présent procédé.

La quantité de palladium déposée sur le support peut varier dans de larges limites. Une quantité comprise entre 1 et 10 % en poids paraît être un compromis acceptable entre les contraintes économiques et l'efficacité recherchée.

La dicarbonylation est conduite également en présence d'un chlorure d'onium quaternaire d'un élément du groupe VB choisi parmi l'azote et le phosphore, ledit élément étant tétracoordonné à des atomes de carbone, l'azote pouvant être coordonné à 2 atomes de phosphore pentavalent.

Les cations onium quaternaire en cause sont bien connus des hommes de l'art qui se réferont utilement à la demande européenne EP-A-0 347 340 incorporée par référence.

De préférence, on recourt à un chlorure d'onium quaternaire dont le cation répond à l'une des formules (I) et (II) ci-après :

I)

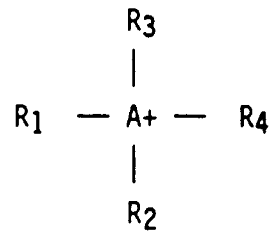

II)

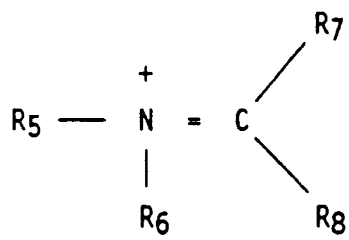

dans lesquelles :

- A représente de l'azote ou du phosphore
- $R_1$, $R_2$, $R_3$, $R_4$ sont identiques ou différents et représentent :
    * un radical alkyle linéaire ou ramifié contenant de 1 à 8 atomes de carbone,
    * un radical aryle contenant de 6 à 10 atomes de carbone, éventuellement substitué par un ou des radicaux alkyles contenant de 1 à 4 atomes de carbone ;
- $R_5$, $R_6$, $R_7$, $R_8$ sont identiques ou différentes et représentent :
    * un radical alkyle linéaire ou ramifié contenant de 1 à 4 atomes de carbone ;
    * les radicaux $R_7$ et $R_8$ pouvant former ensemble un radical alkylène, contenant de 3 à 6 atomes de carbone ;
    * les radicaux $R_6$ et $R_7$ ou $R_6$ et $R_8$ pouvant former ensemble un radical alkylène, alcénylène ou alcadiènylène, contenant 4 atomes de carbone et constituant avec N un hétérocycle azoté.

On recourt avantageusement à un chlorure de phosphonium quaternaire dont le cation répond à la formule (I) ci-avant.

Le chlorure de tétrabutylphosphonium disponible et particulièrement efficace est plus spécialement recommandé.

Le rapport molaire cation onium/palladium est en général compris entre 0,5 et 150, la limite supérieure dudit rapport n'étant dictée que par des considérations économiques. De préférence ledit rapport molaire est compris entre 5 et 150.

Selon une autre caractéristique essentielle de la présente invention, la dicarbonylation est réalisée en présence d'au moins une source de chlorure d'hydrogène.

Par source de chlorure d'hydrogène on entend dans le cadre du présent procédé, HCl gazeux, les solutions aqueuses ou organiques d'HCl et les chlorures organiques capables de libérer HCl dans les conditions réactionnelles.

Parmi les chlorures organiques, on recourt avantageusement aux chlorhydrates d'amine, faciles à manipuler et qui, lorsqu'on souhaite obtenir de manière prépondérante des esters alkyliques de l'acide hexène-3 dioïque-1,6, ne conduisent pas à la formation d'une quantité supplémentaire dudit acide.

Plus spécifiquement, le chlorhydrate d'amine dérive d'une amine répondant à l'une quelconque des formules (III) à (VI) ci-après :

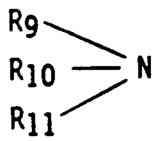

(III)

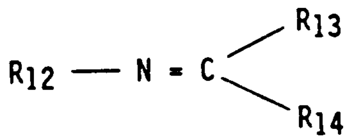

(IV)

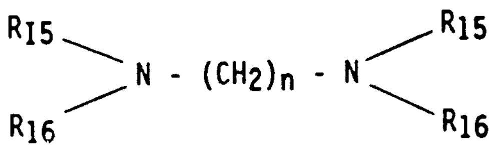

(V)

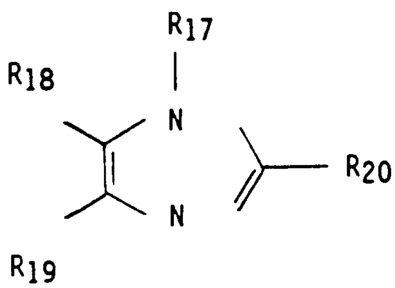

(VI)

dans lesquelles :

- $R_9$ à $R_{11}$ sont identiques ou différents et représentent :
  . un atome d'hydrogène
  . un radical alkyle linéaire ou ramifié contenant de 1 à 16 atomes de carbone, éventuellement substitué par un groupe phényle, hydroxy, halogéno, nitro, alcoxy ou alcoxycarbonyle ;
  . un radical alcényle linéaire ou ramifié contenant de 2 à 12 atomes de carbone, de préférence de 4 à 8 atomes de carbone,
  . un radical aryle contenant de 6 à 10 atomes de carbone, éventuellement substitué par un ou des radicaux alkyles contenant de 1 à 4 atomes de carbone, alcoxy, alcoxycarbonyle ou halogéno ;
  . deux desdits radicaux $R_9$ à $R_{11}$ pouvant former ensemble un radical alkylène, alcénylène ou alcadiènylène, linéaire ou ramifié et contenant de 3 à 6 atomes de carbone ;
- $R_{12}$ à $R_{14}$ sont identiques ou différents et représentent :
  . un radical alkyle linéaire ou ramifié contenant de 1 à 4 atomes de carbone ;
  . les radicaux $R_{13}$ et $R_{14}$ pouvant former ensemble un radical alkylène, contenant de 3 à 6 atomes de carbone ;
  . les radicaux $R_{12}$ et $R_{13}$ (ou $R_{12}$ et $R_{14}$) pouvant former ensemble un radical alkylène, alcénylène ou alcadiènylène, contenant 4 atomes de carbone et constituant avec N un hétérocycle azoté ;
- $R_{15}$ et $R_{16}$ sont identiques ou différents et représentent :
  . un atome d'hydrogène
  . un radical alkyle linéaire ou ramifié contenant de 1 à 4 atomes de carbone ;
  . un radical alcényle linéaire ou ramifié, contenant de 2 à 12 atomes de carbone, de préférence de 4 à 8 atomes de carbone ;
  . n est un nombre entier compris entre 1 et 6 inclus ;

- $R_{17}$ a la signification donnée ci-avant pour $R_9$ à $R_{11}$ ;
- $R_{18}$ à $R_{20}$ sont identiques ou différents et représentent :
  . un atome d'hydrogène ;
  . un radical alkyle linéaire ou ramifié contenant de 1 à 16 atomes de carbone, éventuellement substitué par un groupe phényle, hydroxy, halogéno, nitro, alcoxy ou alcoxycarbonyle ;
  . un radical alcényle linéaire ou ramifié contenant de 2 à 12 atomes de carbone, de préférence de 4 à 8 atomes de carbone.

A titre d'exemples d'amines répondant à la formule (III) on peut citer :

. l'ammoniac,
. les mono-, di- et triméthylamines
. les mono-, di- et triéthylamines
. les mono-, di- et tri- n-propylamines
. les mono-, di- et tributylamines
. les mono-, di- et tri-i-propylamines
. les mono-, di- et tripentylamines
. les mono-, di- et trihexylamines
. les mono-, di- et trioctylamines
. les mono-, di- et tridodécylamines
. la cyclohexylamine
. l'aniline
. la diphénylamine
. la benzylamine
. la triéthanolamine
. la diéthylméthylamine
. la N,N-diméthyléthanolamine
. la N,N-diéthylaniline
. la diéthyl-2,6 aniline
. la N,N-diéthylcyclohexylamine
. la diméthylaminobenzylamine
. la chloro-2-N,N-diméthyléthylamine
. la pyrolidine
. la chloro-2 aniline
. la triéthylènediamine
. la quinuclidine
. la tétrahydro-1,2,3,4 naphtyl-1 amine
. la pipérazine

A titre d'exemples d'amines répondant à la formule (IV) on peut citer :

. la pyridine
. la méthyl-3 pyridine
. la phényl-2 pyridine
. l'indole
. la picoline-2
. le diazabicyclo[5.4.0] undec-7 ène
. le pyrrole
. le méthyl-1 pyrrole
. la quinoline
. la tétrahydro-5,6,7,8 isoquinoléine
. la pyrimidine

A titre d'exemples d'amines répondant à la formule (V) on peut citer :

. la N,N′-diéthyléthylènediamine
. la N,N-diéthyl-N′-méthyléthylènediamine
. la N,N′-diéthyl-1,3 propanediamine
. la N,N,N′,N′-tétraméthyl-1,4 butanediamine
. la N,N,N′,N′-tétraéthylènediamine

A titre d'exemples d'amines répondant à la formule (VI) on peut citer :

. l'imidazole
. le méthyl-1 imidazole.

On recourt avantageusement à un chlorhydrate dérivant d'une amine choisie dans le groupe constitué par la méthylamine, la pyridine, le méthylimidazol, la benzylamine, l'aniline et la chloroaniline.

De préférence, on met en oeuvre un chlorhydrate d'amine tertiaire pour éviter les risques de formation d'amides.

La quantité de chlorhydrate d'amine à mettre en oeuvre dans cadre du procédé selon l'invention peut varier dans de larges limites.

De bons résultats peuvent être observés avec une quantité de chlorhydrate d'amine telle que le rapport molaire chlorhydrate/Pd soit compris entre 0,5 et 100 et, de préférence entre 2 et 20.

La réaction de dicarbonylation peut bien entendu être mise en oeuvre en l'absence ou en présence de solvant exogène au système réactionnel. Lorsqu'on souhaite recourir à des solvants organiques on peut utiliser des solvants aprotiques polaires et basiques tels la N-méthylpyrrolidone-2, la tétraméthylurée et le N,N-diméthylacétamide ou des solvants apolaires et non basiques tels les esters, les hydrocarbures aliphatiques ou cycloaliphatiques saturés, ou les hydrocarbures aromatiques.

Lorsqu'on recourt à un solvant, sa quantité représente au moins 10 % du volume réactionnel ; de bons résultats peuvent être obtenus lorsqu'on en utilise de l'ordre de 20 à 90 % en volume.

La réaction de dicarbonylation est généralement conduite à une température comprise entre 50 et 180 ° C, de préférence entre 80 et 150 ° C, sous une pression d'oxyde de carbone supérieure ou égale à 20 bars et de préférence, inférieure ou égale à 250 bar.

Pour une bonne mise en oeuvre du procédé selon l'invention, la pression d'oxyde de carbone sera de préférence comprise entre 90 et 180 bars.

Des gaz inertes tels l'azote, l'argon ou le gaz carbonique, peuvent être présents à côté de l'oxyde de carbone.

En fin de réaction ou du temps imparti à la réaction, on récupère le diacide et/ou son diester recherché-(s) par tout moyen approprié, par exemple par extraction et/ou distillation.

Les exemples ci-après illustrent l'invention.

EXEMPLES 1 A 5 ; Essai témoin (a) :

Dans un autoclave en acier inoxydable (316 L) de 125 cm3 préalablement purgé à l'argon, on introduit :

- 52 mmol de diméthoxy-1,4 butène-2
- 0,5 mmol de palladium sous forme de palladium déposé sur du charbon actif [0,5 g de Pd/C à 10 % (pds) de palladium]
- 13,6 mmol de chlorure de tétrabutylphosphonium (4 g)
- 5 mmol de chlorhydrate d'amine dont la nature est précisée dans le tableau I ci-après.

L'autoclave est fermé hermétiquement, placé dans un four agité et raccordé à l'alimentation de gaz sous pression. On purge le réacteur à froid avec de l'oxyde de carbone et on le porte à 120 ° C. On régule ensuite la pression à 140 bar. Après réaction, l'autoclave est refroidi et dégazé.

Le mélange réactionnel est alors analysé par chromatographie en phase gazeuse.

Les conditions particulières ainsi que les résultats obtenus figurent au tableau I ci-après dans lequel :

- t représente la durée de réaction en température
- $H_3D$ (%) représente la quantité molaire d'héxène-3 dioate de méthyle formée pour 100 moles de diméthoxy-1,4 butène-2 chargées et

- TT (%) représente le taux de transformation du diméthoxy-1,4 butène-2.

### TABLEAU I

| Réf. | Amine dont dérive le chlorhydrate | t (h) | TT (%) | $H_3D$ (%) |
|---|---|---|---|---|
| 1 | chloroaniline | 2,8 | 100 | 73 |
| 2 | aniline | 2,3 | 100 | 69 |
| 3 | pyridine | 3,5 | 100 | 86 |
| 4 | méthylimidazol | 3,0 | 100 | 86 |
| 5 | benzylamine | 2,4 | 100 | 90 |
| a | néant | 1,5 | 96 | 36 |

Essais témoins (b) à (d) :

Dans l'autoclave et selon le mode opératoire décrits ci-avant on réalise une série d'essais sur une charge renfermant :

- 35 mmol de diméthoxy-1,4 butène-2
- 0,5 mmol de palladium engagé sous forme de palladium déposé sur du charbon actif (10 % pds de palladium)
- 18,5 mmol de chlorhydrate de pyridine

On n'observe aucune formation de produit de dicarbonylation sous 140 bars et dans les conditions particulières indiquées dans le tableau II ci-après :

### TABLEAU II

| Réf | T(°C) | t(h) |
|---|---|---|
| b | 120 | 0,75 |
| c | 140 | 2 |
| d | 150 | 1 |

EXEMPLE 6 :

Dans l'autoclave et selon le mode opératoire décrit ci-avant on réalise un essai sur une charge renfermant :

- 34 mmol de diméthoxy-1,2 butène-3
- 0,5 mmol de palladium sous forme de palladium déposé sur du charbon actif [0,5 g de Pd/C à 10 % (pds) de Pd])
- 13,6 mmol de chlorure de tétrabutylphosphonium (4 g)
- 5 mmol de chlorhydrate de pyridine

Après 3 heures de réaction à 120 °C sous 140 bars, l'autoclave est refoidi et dégazé.

Le mélange réactionnel est analysé par chromatographie liquide à haute performance et chromatographie en phase gazeuse ; puis il est estérifié par du méthanol, au reflux en présence de traces d'acide sulfurique avant d'être de nouveau analysé par chromatographie en phase gazeuse.

Les résultats obtenus sont les suivants :

- Taux de transformation du diméthoxybutène : 100 %.
  Avant estérification, on dose les produits suivants (en mole pour 100 moles de diméthoxybutène chargé) :

| | |
|---|---|
| - Monométhylester de l'acide hexène-3 dioïque-1,6<br>- Hexène-3 dioate de méthyle | = 16 %<br>= 66 % |

Après estérification, on dose :

| | |
|---|---|
| - Hexène-3 dioate de méthyle | = 77 % |

EXEMPLE 7 :

Dans l'autoclave et selon le mode opératoire décrit ci-avant on réalise un essai sur une charge renfermant :

- 35 mmol de diacétoxy-1,2 butène-3
- 0,5 mmol de palladium sous forme de palladium déposé sur du charbon actif [0,5 g de Pd/C à 10 % (pds) de Pd])
- 13,6 mmol de chlorure de tétrabutylphosphonium (4 g)
- 5 mmol de chlorhydrate de pyridine

Après 2,5 heures de réaction à 120 °C sous 140 bars, l'autoclave est refoidi et dégazé.
Le mélange réactionnel est analysé avant et après estérification comme décrit pour l'exemple 6
Les résultats obtenus sont les suivants :

- Taux de transformation du diacétoxybutène : 100 %.
  Avant estérification, on dose les produits suivants (en mole pour 100 moles de diacétoxybutène chargé) :

| | |
|---|---|
| - Acide hexène-3 dioïque-1,6<br>- Monométhylester de l'acide hexène-3 dioïque-1,6<br>- Hexène-3 dioate de méthyle | = 9 %<br>= 24 %<br>= 41 % |

Après estérification, on dose :

| | |
|---|---|
| - Hexène-3 dioate de méthyle | = 66 % |

EXEMPLE 8 :

Dans l'autoclave et selon le mode opératoire décrit ci-avant on réalise un essai sur une charge renfermant :

- 36 mmol de diacétoxy-1,4 butène-2
- 72 mmol d'eau
- 0,5 mmol de palladium sous forme de palladium déposé sur du charbon actif [0,5 g de Pd/C à 5 % (pds) de Pd]
- 13,6 mmol de chlorure de tétrabutylphospnonium (4 g)
- 5 mmol de chlorhydrate de pyridine.

Après 3 heures de réaction à 120 °C sous 140 bar, l'autoclave est refroidi et dégazé.
Le mélange réactionnel est analysé avant et après estérification comme décrit dans l'exemple 6.
Les résultats obtenus sont les suivants :

- Taux de transformation du diacétoxybutène : 100 %
  Avant estérification

| | |
|---|---|
| - Acide hexène-3 dioïque-1,6<br>- monométhylester de l'acide héxène-3 dioïque-1,6<br>- hexène-3 dioate de méthyle | = 47 %<br>= 6 %<br>= 0 % |

Après estérification :

| | |
|---|---|
| - Hexène-3 dioate de méthyle | = 48 % |

EXEMPLE 9 :

Dans l'autoclave et selon le mode opératoire décrit ci-avant on réalise un essai sur une charge renfermant :

- 39 mmol de butènediol-1,4
- 70 mmol de méthanol
- 0,5 mmol de palladium sous forme de palladium déposé sur du charbon actif [0,5 g de Pd/C à 0,5 % (pds) de Pd]
- 13,6 mmol de chlorure de tétrabutylphosphonium (4 g)
- 5 mmol de chlorhydrate de pyridine

Après 2 heures de réaction à 120 °C sous 140 bar, l'autoclave est refroidi et dégazé.

Le mélange réactionnel est estérifié puis analysé comme décrit précédemment.

Les résultats obtenus sont les suivants :

| | |
|---|---|
| - Taux de transformation du butanediol-1,4 | = 100 % |

Après estérification

| | |
|---|---|
| - Hexène-3 dioate de méthyle<br>- Acide hexène-3 dioïque-1,6 | = 50 %<br>= 12 % |

EXEMPLES 10 à 15 :

Dans l'autoclave et selon le mode opératoire décrit ci-avant on réalise une série d'essais sur une charge renfermant :

- 53 mmol de diméthoxy-1,4 butène-2
- 0,5 mmol de palladium engagé sous forme de palladium déposé sur charbon actif (10 % pds)
- du chlorure de tétrabutylphosphonium en quantité variable, précisée dans le tableau III ci-après :
- 5 mmol de chlorhydrate de méthylamine
- de l'adipate de diméthyle : qsp 50 ml sauf indication contraire.

Les conditions particulières ainsi que les résultats obtenus à 120 °C sous 140 bar sont rassemblés dans le tableau III ci-après. Les conventions utilisées y sont les mêmes que pour le tableau I.

TABLEAU III

| Réf. | $PBu_4Cl$ (mmol) | t (h) | TT % | H3D (%) |
|---|---|---|---|---|
| 10 | 3,4 | 4 | 99,5 | 52 |
| 11 | 6,8 | 4 | 100 | 70 |
| 12 | 13,6 | 3 | 99 | 72 |
| 13 | 27 | 4 | 100 | 68 |
| 14 (*) | 6,8 | 4 | 100 | 81 |
| 15 (**) | 6,8 | 3 | 100 | 60 |

(*) essai réalisé avec un volume total de 25 cm3 et une teneur en eau de 0,2 mmol. On note la formation d'une faible quantité (2 %) du monoester méthylique de l'acide hexène-3 dioïque.

(**) essai réalisé avec un volume total de 25 cm3 et une teneur en eau de 6 mmol. On note la formation d'une faible quantité (1 %) du monoester méthylique de l'acide hexène-3 dioïque.

EXEMPLE 16 :

Dans l'autoclave et selon le mode opératoire décrits ci-avant on réalise un essai sur une charge renfermant :

- 35 mmol de diméthoxy-1,4 butène-2
- 0,5 mmol de palladium engagé sous forme de palladium déposé sur charbon actif (10 % pds)
- 13,6 mmol de chlorure de tétrabutylphosphonium
- 5 mmol de chlorhydrate de pyridine
- 10 ml de pentane

Les résultats obtenus après 3,3 heures de réaction à 120 °C sous 140 bar sont les suivants :

- TT (%) = 100
- H3D (%) = 71
- monométhylester de l'acide hexène-3 dioïque-1,6 (%) = 24
- acide hexène-3 dioïque-1,6 (%) = 3

EXEMPLE 17 à 21 :

Dans l'autoclave et selon le mode opératoire décrits ci-avant on réalise une série d'essais sur une charge renfermant :

- 17,8 mmol de diméthoxy-1,2 butène-3
- 0,5 mmol de palladium engagé sous forme de palladium déposé sur charbon actif à 10 % (pds) de Pd (sauf mention contraire)
- 14 mmol de chlorure de tétrabutylphosphonium,
- 2 mmol d'acide chlorhydrique en solution aqueuse (10 N) (sauf mention contraire)
- un solvant hydrocarboné dont la nature et la quantité sont indiquées dans le tableau IV ci-après.

En fin de réaction on dose le mélange réactionnel par chromatographie en phase gazeuse.

Les conditions particulières ainsi que les résultats obtenus à 120 °C sous 140 bar figurent dans le tableau IV ci-après, dans lequel :

- t(h) désigne la durée de réaction exprimée en heure :
- H3D (%) à la signification donnée précédemment
- MME (%) représente la quantité molaire de monométhylester de l'acide hexène-3 dioïque-1,6 pour 100 moles de diméthoxy-1,2 butène-3 chargées,
- ACIDE (%) représente la quantité molaire d'acide hexène-3 dioïque-1,6 pour 100 moles de diméthoxy-1,2 butène-3 chargées.

TABLEAU IV

| Réf. | Pd | Solvant | | t | TT | H3D | MME | ACIDE |
|---|---|---|---|---|---|---|---|---|
| | mmol | nature | ml | (h) | (%) | (%) | (%) | (%) |
| 17 (*) | 0,5 | cyclohexane | 30 | 1 | 98 | 14 | 12 | 47 |
| 18 | 0,5 | " | " | 1 | 100 | 32 | 36 | 27 |
| 19 | 0,5 | pentane | 60 | 1,4 | 94 | 26 | 24 | 33 |
| 20 | 0,2 | cyclohexane | 30 | 2 | 99 | 40 | 35 | 15 |
| 21 (**) | 0,25 | pentane | 60 | 1,7 | 76 | 14 | 12 | 32 |

(*) essai réalisé avec 5 mmol d'HCl (10N)

(**) essai réalisé avec 7 mmol de $PBu_4Cl$

## Revendications

1. Procédé de dicarbonylation linéaire de butènes difonctionnalisés en présence d'une quantité catalytiquement efficace de palladium et d'un chlorure d'onium quaternaire d'un élément du groupe VB choisi parmi l'azote et le phosphore, ledit élément étant tétracoordonné à des atomes de carbone, l'azote pouvant être coordonné à 2 atomes de phosphore pentavalent, en phase liquide, à température élevée et sous une pression supérieure à la pression atmosphérique, caractérisé en ce que
   a) le palladium est engagé au moins partiellement sous forme de palladium à l'état d'oxydation nul et
   b) la réaction est conduite également en présence d'au moins une source de chlorure d'hydrogène.

2. Procédé selon la revendication 1, caractérisé en ce que le palladium est engagé sous forme de palladium métallique déposé sur un support.

3. Procédé selon la revendication 2, caractérisé en ce que le support est du charbon actif.

4. Procédé selon l'une quelconque des revendications précédentes, caractérisé en ce que la source de chlorure d'hydrogène est choisie dans le groupe constitué par HCl gazeux, les solutions aqueuses d'HCl, les solutions organiques d'HCl et les chlorures organiques capables de libérer HCl dans les conditions réactionnelles;

5. Procédé selon l'une quelconque des revendications précédentes caractérisé en ce que le chlorhydrate dérive d'une amine répondant à l'une quelconque des formules (III) à (VI) ci-après :

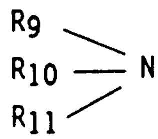

(III)

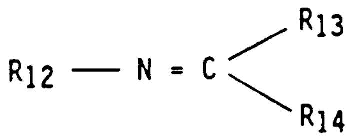

(IV)

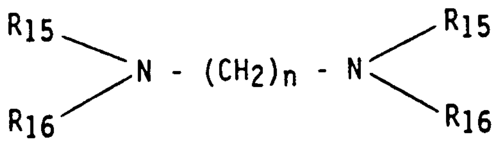

(V)

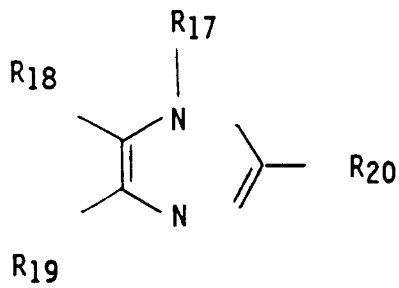

(VI)

dans lesquelles :

- $R_9$ à $R_{11}$ sont identiques ou différents et représentent :
  . un atome d'hydrogène
  . un radical alkyle linéaire ou ramifié contenant de 1 à 16 atomes de carbone, éventuellement substitué par un groupe phényle, hydroxy, halogéno, nitro, alcoxy ou alcoxycarbonyle ;
  . un radical alcényle linéaire ou ramifié contenant de 2 à 12 atomes de carbone,
  . un radical aryle contenant de 6 à 10 atomes de carbone, éventuellement substitué par un ou des radicaux alkyles contenant de 1 à 4 atomes de carbone, alcoxy, alcoxycarbonyle ou halogéno ;
  . deux desdits radicaux $R_9$ à $R_{11}$ pouvant former ensemble un radical alkylène, alcénylène ou alcadiènylène, linéaire ou ramifié et contenant de 3 à 6 atomes de carbone ;
- $R_{12}$ à $R_{14}$ sont identiques ou différents et représentent :
  . un radical alkyle linéaire ou ramifié contenant de 1 à 4 atomes de carbone ;
  . les radicaux $R_{13}$ et $R_{14}$ pouvant former ensemble un radical alkylène, contenant de 3 à 6 atomes de carbone ;
  . les radicaux $R_{12}$ et $R_{13}$ (ou $R_{12}$ et $R_{14}$) pouvant former ensemble un radical alkylène, alcénylène ou alcadiènylène, contenant 4 atomes de carbone et constituant avec N un hétérocycle azoté ;
- $R_{15}$ et $R_{16}$ sont identiques ou différents et représentent :
  . un atome d'hydrogène
  . un radical alkyle linéaire ou ramifié contenant de 1 à 4 atomes de carbone ;
  . un radical alcényle linéaire ou ramifié, contenant de 2 à 12 atomes de carbone ;
  . n est un nombre entier compris entre 1 et 6 inclus ;
- $R_{17}$ a la signification donnée ci-avant pour $R_9$ à $R_{11}$ ;
- $R_{18}$ à $R_{20}$ sont identiques ou différents et représentent :
  . un atome d'hydrogène ;
  . un radical alkyle linéaire ou ramifié contenant de 1 à 16 atomes de carbone, éventuellement substitué par un groupe phényle, hydroxy, halogéno, nitro, alcoxy ou alcoxycarbonyle ;

. un radical alcényle linéaire ou ramifié contenant de 2 à 12 atomes de carbone.

6. Procédé selon l'une quelconque des revendications précédentes caractérisé en ce que l'amine dont dérive le chlorhydrate est choisie dans le groupe constitué par la méthylamine, la pyridine, le méthylimidazole, la benzylamine, l'aniline et la chloroaniline.

7. Procédé selon l'une quelconque des revendications précédentes caractérisé en ce que le chlorhydrate dérive d'une amine tertiaire.

8. Procédé selon l'une quelconque des revendications précédentes caractérisé en ce que la réaction est conduite dans un solvant choisi parmi les hydrocarbures.

9. Procédé selon l'une quelconque des revendications précédentes caractérisé en ce que le rapport molaire chlorhydrate d'amine/Pd est compris entre 0,5 et 100.

10. Procédé selon l'une quelconque des revendications précédentes caractérisé en ce que la concentration du palladium dans le milieu réactionnel est comprise entre $10^{-3}$ et 1 mol/l.

11. Procédé selon l'une quelconque des revendications précédentes, caractérisé en ce que le rapport molaire chlorure d'onium quaternaire/Pd est compris entre 0,5 et 150 et de préférence entre 5 et 150.

12. Procédé selon l'une quelconque des revendications précédentes, caractérisé en ce que le chlorure d'onium quaternaire est le chlorure de tétrabutylphosphonium.

13. Procédé selon l'une quelconque des revendications précédentes, caractérisé en ce que la température de réaction est comprise entre 50 et 180 ° C et, de préférence, entre 80 et 150 ° C.

14. Procédé selon l'une quelconque des revendications précédentes, caractérisé en ce que la pression est supérieure ou égale à 20 bar et, de préférence, inférieure ou égale à 250 bar.

15. Procédé selon l'une quelconque des revendications précédentes, caractérisé en ce que la pression est comprise entre 90 et 180 bar.

**Claims**

1. Process for the linear dicarbonylation of difunctionalized butenes in the liquid phase, at high temperature and at a pressure greater than atmospheric pressure, in the presence of a catalytically effective amount of palladium and of a quaternary onium chloride of an element of group Vb chosen from nitrogen and phosphorus, the said element being tetracoordinated to carbon atoms, it being possible for the nitrogen to be coordinated to 2 pentavalent phosphorus atoms, characterized in that
   a) the palladium is charged at least partially in the form of palladium in the zero oxidation state and
   b) the reaction is also carried out in the presence of at least one hydrogen chloride source.

2. Process according to claim 1, characterized in that the palladium is charged in the form of metallic palladium deposited on a support.

3. Process according to claim 2, characterized in that the support is active charcoal.

4. Process according to any one of the preceding claims, characterized in that the hydrogen chloride source is chosen from the group consisting of gaseous HCl, aqueous HCl solutions, organic HCl solutions and organic chlorides capable of releasing HCl under the reaction conditions.

5. Process according to any one of the preceding claims, characterized in that the hydrochloride derives from an amine corresponding to any one of the formulae (III) to (VI) hereinbelow:

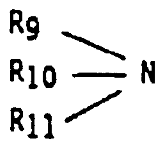

(III)

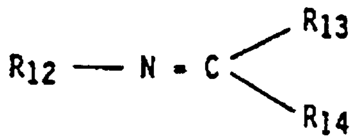

(IV)

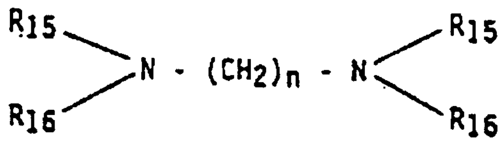

(V)

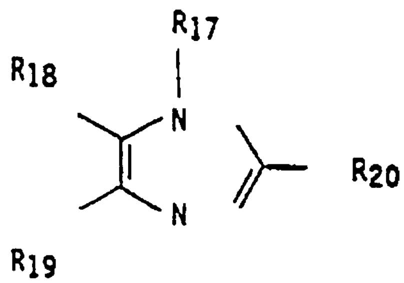

(VI)

i n
which:

- $R_9$ to $R_{11}$ are identical or different and represent:
  - a hydrogen atom;
  - a linear or branched alkyl radical containing from 1 to 16 carbon atoms and optionally substituted by a phenyl, hydroxyl, halo, nitro, alkoxy or alkoxycarbonyl group;
  - a linear or branched alkenyl radical containing from 2 to 12 carbon atoms;
  - an aryl radical containing from 6 to 10 carbon atoms and optionally substituted by one or more alkyl, alkoxy, alkoxycarbonyl or halo radicals, the alkyl radicals containing from 1 to 4 carbon atoms;
  - it being possible for two of the said radicals $R_9$ to $R_{11}$ to form together an alkylene, alkenylene or alkadienylene radical which is linear or branched and which contains from 3 to 6 carbon atoms;
- $R_{12}$ to $R_{14}$ are identical or different and represent:
  - a linear or branched alkyl radical containing from 1 to 4 carbon atoms;
  - it being possible for the radicals $R_{13}$ and $R_{14}$ to form together an alkylene radical containing from 3 to 6 carbon atoms;
  - it being possible for the radicals $R_{12}$ and $R_{13}$ (or $R_{12}$ and $R_{14}$) to form together an alkylene, alkenylene or alkadienylene radical containing 4 carbon atoms and constituting, with N, a nitrogenous heterocycle;
- $R_{15}$ and $R_{16}$ are identical or different and represent:
  - a hydrogen atom;
  - a linear or branched alkyl radical containing from 1 to 4 carbon atoms;
  - a linear or branched alkenyl radical containing from 2 to 12 carbon atoms;
  - n is an integer between 1 and 6 inclusive;
  - $R_{17}$ has the meaning given hereinabove for $R_9$ to $R_{11}$;
- $R_{18}$ to $R_{20}$ are identical or different and represent:

- a hydrogen atom;
- a linear or branched alkyl radical containing from 1 to 16 carbon atoms and optionally substituted by a phenyl, hydroxyl, halo, nitro, alkoxy or alkoxycarbonyl group;
- a linear or branched alkenyl radical containing from 2 to 12 carbon atoms.

**6.** Process according to any one of the preceding claims, characterized in that the amine from which the hydrochloride derives is chosen from the group consisting of methylamine, pyridine, methylimidazole, benzylamine, aniline and chloroaniline.

**7.** Process according to any one of the preceding claims, characterized in that the hydrochloride derives from a tertiary amine.

**8.** Process according to any one of the preceding claims, characterized in that the reaction is carried out in a solvent chosen from the hydrocarbons.

**9.** Process according to any one of the preceding claims, characterized in that the amine hydrochloride/Pd molar ratio is between 0.5 and 100.

**10.** Process according to any one of the preceding claims, characterized in that the concentration of the palladium in the reaction medium is between $10^{-3}$ and 1 mol/l.

**11.** Process according to any one of the preceding claims, characterized in that the quaternary onium chloride/Pd molar ratio is between 0.5 and 150 and preferably between 5 and 150.

**12.** Process according to any one of the preceding claims, characterized in that the quaternary onium chloride is tetrabutylphosphonium chloride.

**13.** Process according to any one of the preceding claims, characterized in that the reaction temperature is between 50 and 180 °C and preferably between 80 and 150 °C.

**14.** Process according to any one of the preceding claims, characterized in that the pressure is greater than or equal to 20 bar and preferably less than or equal to 250 bar.

**15.** Process according to any one of the preceding claims, characterized in that the pressure is between 90 and 180 bar.

## Patentansprüche

**1.** Verfahren zur linearen Dicarbonylierung von difunktionellen Butenen in Gegenwart einer katalytisch wirksamen Menge von Palladium und von einem quaternären Oniumchlorid eines Elements der Gruppe VB, ausgewählt unter Stickstoff und Phosphor, wobei dieses Element tetrakoordiniert an Kohlenstoffatomen ist, der Stickstoff an 2 fünfwertigen Phosphoratomen koordiniert sein kann, in flüssiger Phase bei erhöhter Temperatur und unter einem höheren Druck als dem atmosphärischen Druck, dadurch gekennzeichnet, daß

a) das Palladium zumindest teilweise in Form von Palladium im Oxidationszustand Null eingesetzt wird, und

b) die Umsetzung auch in Anwesenheit zumindest einer Quelle für Chlorwasserstoff durchgeführt wird.

**2.** Verfahren gemäß Anspruch 1,
dadurch gekennzeichnet, daß
das Palladium in Form von metallischem, auf einem Träger abgeschiedenem Palladium eingesetzt wird.

**3.** Verfahren gemäß Anspruch 2,
dadurch gekennzeichnet, daß
der Träger Aktivkohle ist.

**4.** Verfahren gemäß einem der vorhergehenden Ansprüche, dadurch gekennzeichnet, daß
die Quelle für Chlorwasserstoff unter gasförmigem HCl, Wäßrigen HCl-Lösungen, organischen HCl-Lösungen und organischen Chloriden, die im Stande sind, unter den Reaktionsbedingungen HCl freizusetzen, ausgewählt wird.

**5.** Verfahren gemäß einem der vorhergehenden Ansprüche, dadurch gekennzeichnet, daß
das sich von einem Amin ableitende Hydrochlorid einer der nachstehenden Formeln (III) bis (VI) entspricht:

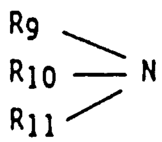

(III)

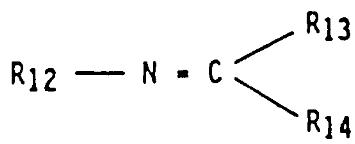

(IV)

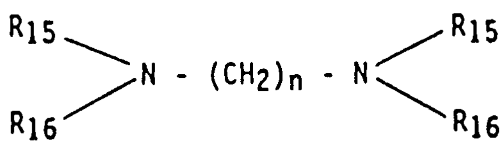

(V)

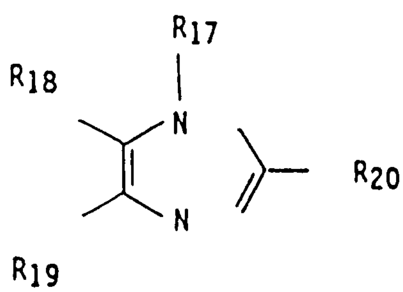

(VI)

worin:

- $R_9$ bis $R_{11}$ identisch oder verschieden sind und bedeuten:
  . ein Wasserstoffatom,
  . einen linearen oder verzweigten Alkylrest mit 1 bis 16 Kohlenstoffatomen, der gegebenenfalls durch eine Phenyl-, Hydroxy-, Halogen-, Nitro-, Alkoxy- oder Alkoxycarbonylgruppe substituiert ist,
  . einen linearen oder verzweigten Alkenylrest mit 2 bis 12 Kohlenstoffatomen,
  . einen Arylrest mit 6 bis 10 Kohlenstoffatomen, der gegebenenfalls durch einen oder mehreren Alkylreste mit 1 bis 4 Kohlenstoffatomen, Alkoxy, Alkoxycarbonyl oder Halogen substituiert ist,
  . zwei der Reste $R_9$ bis $R_{11}$ können hierbei gemeinsam einen linearen oder verzweigten Alkylen-, Alkenylen- oder Alkadienylenrest mit 3 bis 6 Kohlenstoffatomen bilden,
- $R_{12}$ bis $R_{14}$ identisch oder verschieden sind und bedeuten:
  . einen linearen oder verzweigten Alkylrest mit 1 bis 4 Kohlenstoffatomen,
  . die Reste $R_{13}$ und $R_{14}$ können hierbei gemeinsam einen Alkylenrest mit 3 bis 6 Kohlenstoffatomen bilden,
  . die Reste $R_{12}$ und $R_{13}$ (oder $R_{12}$ und $R_{14}$) können hierbei gemeinsam einen Alkylen-, Alkenylen- oder Alkadienylenrest mit 4 Kohlenstoffatomen bilden und mit N einen Stickstoffhal-

tigen Heterocyclus darstellen,

- $R_{15}$ und $R_{16}$ identisch oder verschieden sind und bedeuten:
  . ein Wasserstoffatom,
  . einen linearen oder verzweigten Alkylrest mit 1 bis 4 Kohlenstoffatomen,
  . einen linearen oder verzweigten Alkenylrest mit 2 bis 12 Kohlenstoffatomen,
  . n für eine ganze Zahl zwischen 1 und 6 einschließlich steht,
- $R_{17}$ die vorstehend für $R_9$ bis $R_{11}$ angegebene Bedeutung besitzt,
- $R_{18}$ bis $R_{20}$ identisch oder verschieden sind und bedeuten:
  . ein Wasserstoffatom,
  . einen linearen oder verzweigten Alkylrest mit 1 bis 16 Kohlenstoffatomen, der gegebenenfalls durch eine Phenyl-, Hydroxy-, Halogen-, Nitro-, Alkoxy- oder Alkoxycarbonylgruppe substituiert ist,
  . einen linearen oder verzweigten Alkenylrest mit 2 bis 12 Kohlenstoffatomen.

**6.** Verfahren gemäß einem der vorhergehenden Ansprüche, dadurch gekennzeichnet, daß das Amin, von dem sich das Hydrochlorid ableitet, unter Methylamin, Pyridin, Methylimidazol, Benzylamin, Anilin und Chloranilin ausgewählt wird.

**7.** Verfahren gemäß einem der vorhergehenden Ansprüche, dadurch gekennzeichnet, daß das Hydrochlorid sich von einem tertiären Amin ableitet.

**8.** Verfahren gemäß einem der vorhergehenden Ansprüche, dadurch gekennzeichnet, daß die Umsetzung in einem unter Kohlenwasserstoffen ausgewählten Lösungsmittel durchgeführt wird.

**9.** Verfahren gemäß einem der vorhergehenden Ansprüche, dadurch gekennzeichnet, daß das Molverhältnis Aminhydrochlorid/Pd zwischen 0,5 und 100 liegt.

**10.** Verfahren gemäß einem der vorhergehenden Ansprüche, dadurch gekennzeichnet, daß die Konzentration des Palladiums in dem Reaktionsmilieu zwischen $10^{-3}$ und 1 Mol/l liegt.

**11.** Verfahren gemäß einem der vorhergehenden Ansprüche, dadurch gekennzeichnet, daß das Molverhältnis quaternäres Oniumchlorid/Pd zwischen 0,5 und 150 und vorzugsweise zwischen 5 und 150 liegt.

**12.** Verfahren gemäß einem der vorhergehenden Ansprüche, dadurch gekennzeichnet, daß das quaternäre Oniumchlorid Tetrabutylphosphoniumchlorid ist.

**13.** Verfahren gemäß einem der vorhergehenden Ansprüche, dadurch gekennzeichnet, daß die Reaktionstemperatur zwischen 50 und 180 °C und vorzugsweise zwischen 80 und 150 °C liegt.

**14.** Verfahren gemäß einem der vorhergehenden Ansprüche, dadurch gekennzeichnet, daß der Druck höher oder gleich 20 bar und vorzugsweise niedriger oder gleich 250 bar beträgt.

**15.** Verfahren gemäß einem der vorhergehenden Ansprüche, dadurch gekennzeichnet, daß der Druck zwischen 90 und 180 bar liegt.